# EUROPEAN PATENT SPECIFICATION

(11) **EP 2 696 845 B1**
(45) Date of publication and mention of the grant of the patent: **20.02.2019**
(21) Application number: 12726180.8
(22) Date of filing: 13.04.2012
(51) Int. Cl.: A61K 8/87, A61K 8/55, A61K 8/73, A61Q 5/00, A61Q 5/12, A61Q 7/00, A61Q 19/10, A61Q 5/02

(54) **SYSTEM FOR PROLONGED RELEASE OF COSMETIC AGENTS**
SYSTEM ZUR ZEITVERZÖGERTEN FREIGABE KOSMETISCHER AGENTIEN
SYSTÈME POUR LA LIBÉRATION PROLONGÉE D'AGENTS COSMÉTIQUES

(30) Priority: 14.04.2011 IT MI20110644
(43) Date of publication of application: 19.02.2014
(73) Proprietor: Giuliani S.p.A., 20129 Milano (IT)
(72) Inventor: GIULIANI, Giammaria, I-20123 Milano (IT); BENEDUSI, Anna, I-20124 Milano (IT); BARONI, Sergio, I-24030 Villa d'Adda (IT); MASCOLO, Antonio, I-20126 Milano (IT)
(74) Representative: Coppo, Alessandro
(86) International application number: PCT/IB2012/051826
(87) International publication number: WO 2012/140609

(56) References cited:
- DE-A1- 19 901 484
- JP-A- 2008 266 240
- US-A- 5 723 146
- US-A1- 2002 155 072
- US-A1- 2007 189 980
- US-A1- 2007 248 658
- Tara E. Gottschalck et. al: "International Cosmetic Ingredient Dictionary and Handbook, 13th edition", 2010, Personal Care Products Council, XP002669049, vol. 2, page 2414, Entry "POLYURETHANE-26"
- DATABASE GNPD [Online] Mintel; August 2010 (2010-08), "Refreshing Nourishing Emulsion S", XP002669047, Database accession no. 1415631
- Tara E. Gottschalck et al.: "International Cosmetic Ingredient Dictionary and Handbook, 13th edition", 2010, Personal Care Products Council, XP002669048, vol. 2, page 2412, Entry "POLYURETHANE-8"

## Description

### FIELD OF THE INVENTION

The present invention concerns a system for the prolonged release of cosmetic substances.

The present invention origins in the field of cosmetics and specifically in the field of systems for the release of agents for the cosmetic treatment of hair and skin. Specifically, the present invention concerns systems and phospholipid-based compositions for the prolonged release of cosmetic agents or active substances for keratinic structures in the human body, such as hair and skin.

### STATE OF THE ART

Cosmetic products and preparations for personal care and grooming contain agents or substances suitable for improving the cosmetic and aesthetic properties of the external tissues of the human body, in particular keratinic structures, such as skin, hair and nails.

Generally, cosmetic products do not bind to the keratinic structures of the body in a lasting manner, and are easily removed with each wash using water. Consequently, these products require frequent application to the keratinic structures of the body in order to continue to exercise the desired cosmetic action.

Cosmetic hair-care products are currently available on the market that are not washed away following washing with water and there are likewise known additive substances that are intended to bind to the keratinic structures of the human body, particularly to hair, in a long-lasting manner, and to prolong the release of cosmetic agents to these structures.

Some of these substances are of protein origin and include single-chain or branched peptides that have a tendency to bind to other protein-based structures, particularly the keratinic structures of the human body.

However, use of these peptides in the formulation of cosmetic preparations has not found broad application, since the binding force of an individual peptide is not always sufficient to guarantee an adequate binding effect and furthermore, in many cases these products are costly or difficult to prepare.

Likewise, in the field of hair-care, preparations for the treatment of hair are known that include binding agents in their formulation that are intended to increase their persistence time and the release of the cosmetic preparation.

These agents usually bind due to affinity for the protein component of hair or the scalp, or through the formation of electrostatic bonds.

In cosmetic formulations for hair, binding agents are also used that have free cationic groups, such as for example, certain cationic surfactants and cationic polymers.

The use of cationic polymers in cosmetic preparations for the treatment of hair is not however entirely satisfactory, since these agents have a tendency to make hair heavy, shortening washing times, with consequent washing away of the cosmetic product.

Furthermore, deposition of cosmetic active substances on hair is highly influenced by pH. When pH values are low, as is the case with numerous preparations for hair-care use, the binding capacity of the cationic polymer is greatly reduced whereby, under such conditions, these agents no longer exert any binding effect and the cosmetic agent is easily removed from the site of action.

In the field of cosmetics, and in particular the hair-care sector, attempts are also known to prepare formulations with prolonged cosmetic action by means of the incorporation of phospholipids, substances capable of attaching to keratinic structures due to their amphipathic characteristics. However, again in this case, it has been observed that their incorporation into cosmetic preparations for hair-care use tends to make hear greasy more rapidly, and to bind dust-like materials present in the air, making the hair heavy and sticky, even just a short period of time after application.

Again in this case, the consequence is greater frequency of washing, with the cosmetic product applied being washed away.

US 2002/155072 A1 discloses the use of film-forming, water soluble or water-dispersible polyurethanes for improving the water resistance of cosmetic compositions containing, for example, a water-soluble UV filter as a cosmetically active ingredient.

DE 199 01 484 A1 discloses the use of fluorinated (poly-)urethanes for improving the wash resistance of (semi-)temporary hair dyes.

US 5 723 146 A discloses pharmaceutical preparation preferably in the form of lotions, gel, ointment wherein the active ingredient is encapsulated in a liposome especially phospholipids.

JP 2008 266240 A discloses a hair cosmetic composition which can ensure a moisture-retaining effect based on the compounding of a cationized hyaluronic acid or its analogue, hair gloss and the like. The composition contains (A) a cationized hyaluronic acid prepared by modifying the carboxy groups of glucuronic acids composing the hyaluronic acid with a specific quaternary ammonium-containing modifying group, or an analogue whose functional groups form salts or are modified and (B) at least one selected from dimethiconol and amino-modified silicones.

US 2007/189980 A1 and US 2007/248658 A1 disclose cosmetic compositions exhibiting a sustained release of the active ingredient contained therein due to the presence of polymeric polyurethane.

In the article "Refreshing Nourishing Emulsion S" available from Database GNPD Intel, August 2010 it is disclosed a skin whitening cosmetic containing, amongst other ingredients, a phospholipid, hyaluronic acid and a cationic polyurethane.

At present, the need is therefore felt to provide cosmetic preparations or systems, that when applied to the keratinic structures of the human body, remain in situ in a persistent manner, prolonging the cosmetic action of the agents contained.

There is also the need to provide cosmetic systems that bind tightly to the keratinic structures of the human body, such as for example the hair stalk, and that under certain conditions, persist even to subsequent washing of said structures with water.

### SUMMARY

The applicant of the present invention has found that by combining a phospholipid component with hyaluronic acid or a derivative thereof and a cationic polyurethane, a phospholipid-based system is obtained that binds tightly to keratinic structures of the skin and allows the prolonged release of cosmetic agents.

In accordance with a first aspect of the present invention, a system for the release of cosmetic agents is thus provided, characterised in that it comprises
i) at least one phospholipid (phospholipid component),
ii) 2-hydroxy-3-(N,N,N-trimethylammonium) propyl chloride,
iii) a cationic polyurethane derivative being a perfluoropolyether-polyurethane (PFPE-PU) copolymer comprising a perfluoropolyethereal (Rf) chain having the structure:

   -(CF₂-CF₂O)p-(CF₂O)q-

   wherein p/q = 0.5 - 3.0,
   wherein said perfluoropolyethereal (Rf) chain has a molecular weight of 500 to 4000, preferably of 1000 to 2000 and more preferably of 1400 to 1600,
   in cosmetically acceptable amounts.

In certain embodiments, the system of the invention is a phospholipid-based system and comprises a plurality of phospholipids in variable ratios to one another.

In certain embodiments, the phospholipid system disclosed herein comprises phospholipids obtained from plant or animal raw materials.

In certain embodiments, the system comprises one or more lecithins, wherein said lecithins are obtained from plant raw materials, such as for example Glycine max L., *Helianthus annuus, Brassica carinata* etc., or from animal raw materials, for example egg-yolk.

In certain embodiments, the phospholipid has a diacylglyceride structure (glycerophospholipid) or is a phosphosphingolipid or mixtures thereof.

In certain embodiments, the system of the invention comprises a phospholipid component selected from phosphatidylcholine, phosphatidylserine, phosphatidylethanolamine, phosphatidylinositol, dipalmitoylphosphatidylcholine, lysophospholipids, zwitterionic phospholipids, sphingomyelin, sphingolipids, glycerophospholipids, sphingoglycolipids or mixtures thereof.

Hyaluronic acid 2-hydroxy-3-(N,N,N-trimethylammonium)propyl chloride is another important component of the phospholipid-based system of the present disclosure.

Typically, the hyaluronic acid of the present disclosure is a polysaccharide formed by alternating units of glucuronic acid and N-acetyl glucosamine or a derivative thereof.

The hyaluronic acid derivative used is the 2-hydroxy-3-(N,N,N-trimethylammonium) propyl chloride derivative.

This derivative preserves the basic characteristics of hyaluronic acid, namely the capacity to coordinate a high number of water molecules and fully perform the surface hydrating function typical of these high molecular weight hydrophilic polymers.

The other important component of the system of the invention is a cationic polyurethane derivative as defined in claim 1.

According to certain aspects of the present disclosure, the term cationic polyurethane means a polyurethane containing at least one quaternary nitrogen atom and/or at least one nitrogen atom that can be quaternized/protonated.

The cationic polyurethane comprises a perfluoropolyether-polyurethane (PFPE-PU) copolymer.

Typically, said perfluoropolyether-polyurethane (PFPE-PU) may be schematized as a series of linear chains, the structure of which alternates two types of "chains" with characteristics very different from one another and that thus stabilise different interactions with the substances present in solution in the system of the invention. Typically, the non-fluorinated hydrophilic portions alternate with fluorinated hydrophobic portions giving the copolymer significant water-repellency. The wide inter-chain spaces that form due to the repulsion exerted by the fluorinated portions on the adjacent chains give rise to "wide meshes" which represent water vapour elimination routes, giving the copolymer marked non-occlusiveness characteristics. The hydrophilic portions have a positive charge which mediates anchoring to skin and hair.

Also disclosed herein is the use of a system of the type previously described to change the profile of release of a cosmetic agent to the epidermis or to a keratinic structure of the human body.

Within the scope of the invention, the term system for or suitable for changing the profile of release of a cosmetic agent to the epidermis means a system that modifies or prolongs the release time of a cosmetic agent contained within a cosmetic composition, compared to a composition with the same formulation wherein said system is absent, increasing the adhesion to keratinic structures, for example skin, hair or scalp. This increased adhesion to keratinic structures, increases the resistance to the action of rinsing of the cosmetic compositions containing the system of the invention, compared to those wherein it is absent.

In accordance with a second aspect of the invention, a cosmetic composition is provided comprising a system of the type previously described, at least one cosmetically active ingredient and one or more excipients.

According to certain embodiments, the cosmetic composition of the invention is a composition for use as a non-rinse or leave-on composition, suitable for applying to hair and/or the scalp.

The cosmetic composition containing the system of the invention reduces the frequency of the cosmetic treatment and consequently reduces the exposure to additives, preservatives, perfumes and enhancers commonly used in cosmetic formulations, improving the safety profile of the cosmetic product.

### DETAILED DESCRIPTION OF THE INVENTION

The Applicant has found that by associating components i) - iii) previously described, a system is obtained for the prolonged release of cosmetic agents that can be applied in the preparation of cosmetic products that remain *in situ* after application.

In particular, the phospholipid-based system of the invention modulates the kinetic of release of cosmetic agents or functional substances to which the system is added, prolonging the cosmetic efficacy.

In certain embodiments, one or more cosmetic agents are added to the system of the invention, and the resulting formulation is used as it is for cosmetic applications.

In other embodiments, the system of the invention is incorporated in a cosmetic composition comprising one or more cosmetic agents and excipients suitable for cosmetic formulations (use in association).

It has been found that following the application of the cosmetic composition containing the phospholipid system, evaporation of the solvent(s) contained in the composition occurs, with the formation of a complex polymeric matrix film or gel that alters the release kinetics of the cosmetic active substances or the functional substances present in the composition. This film remains stuck to the treated keratinic surface and possesses a certain degree of resistance to the washing-away action of water.

In certain embodiments, the system of the invention is incorporated in a cosmetic composition for application to the scalp and/or hair. In such cases, the presence of the system allows the cosmetic composition to remain anchored or adhered to keratinic structures, such as the hair stem, without making the structure heavy, and increasing its resistance to washing, prolonging the cosmetic effects. Typically, in the system of the invention, the phospholipidic component establishes bonds with the lipids of sebum and with the phospholipids in the cell membranes of the superficial layers of the epidermis or with the lipophilic domains of skin or hair keratin, increasing the affinity of the cosmetic composition to which it is added to skin and cutaneous parts.

According to one aspect of the present disclosure, the use of a system as described is provided to make a cosmetic agent adherent to epidermis or to a keratinic structure of the human body.

Also disclosed herein is the use of a system as described or claimed to increase the resistance of a cosmetic composition applied to a keratinic structure, such as skin and hair, to rinsing with water (washing away). Specifically, a cosmetic composition containing the system of the invention has greater resistance to washing with water, compared to a composition with the same formulation void of said system.

In certain embodiments, the phospholipid component of the system of the invention comprises or essentially consists of soya lecithin and/or the phospholipid fractions thereof. For example, it may be used the phospholipid mixture obtained by means of degumming the oily fraction extracted from soya seeds. Typically, this phospholipid mixture comprises glycerophospholipids of sn-glycerol-3-phosphate such as phosphatidylcholine, phosphatidylinositol, phosphatidylethanolamine, phosphatidic acid.

In certain embodiments, soya lecithin is used, which comprises the following phospholipid components:
70-85% 3-sn-phosphatidylcholine
3-10% 3-sn-Lysophosphatidylcholine
3-10% Phosphatidylethanolamine
3-10% Phosphatidic acid.

In other embodiments, a soya lecithin is used with a phosphatidylcholine content not lower than 90% w/w and with no more than 6% lysophosphatidylcholine. According to further embodiments, a soya lecithin is used with a phosphatidylcholine content of no less than 94-102% and with no more than 4% lysophosphatidylcholine.

According to certain embodiments, in the system of the invention, the phospholipid is present in amounts of 0.005 to 5% by weight, with respect to the total weight of the system.

The polymer components of the system of the invention, along with the phospholipid, contribute towards altering the release kinetics of the cosmetic agents, and at the same time increase the skin and hair adhesive properties, slowing removal of the system or the cosmetic compositions incorporated following washing.

One of the polymer components of the system is hyaluronic acid 2-hydroxy-3-(N,N,N-trimethylammonium)propyl chloride.

The Applicant has found that the hyaluronic acid 2-hydroxy-3 (N,N,N trimethylammonium) propyl chloride derivative possesses high adhesion properties, despite maintaining the basic characteristics of hyaluronic acid, namely the capacity to coordinate a high number of water molecules and perform the surface hydrating function. On chemical analysis, this derivative is a heteropolysaccharide consisting of two basic monomers, glucuronic acid and N-acetylglucosamine. The presence of positively charged groups in this polymer provides skin and hair adhesion properties, making its removal more difficult following washing.

Certain tests have then demonstrated that thanks to its positive charge, the hyaluronic acid, 2-hydroxy-3(N,N,N trimethylammonium) propyl chloride derivative is in itself capable of anchoring itself to skin and hair, in a much stronger manner than the case of hyaluronic acid of the same molecular weight. According to certain embodiments of the phospholipid-based system of the invention, hyaluronic acid or the previously identified derivative thereof, is present in quantities comprised between 0.002 and 0.5% by weight, with respect to the total weight of the system.

The presence of the polyurethane component in the system of the invention synergically increases the capacity of hyaluronic acid or the derivatives thereof to adhere to skin or to keratinic structures.

The cationic polyurethane derivative is obtained by reacting a perfluoropolyether dialcohol with a linear structure (OH-CH₂-Rf-CH₂-OH) with isophorone isocyanate, for example by operating with an excess of isophorone isocyanate, which is then eliminated by reacting with a solvent, for example hexane, followed by reaction with a tertiary amine containing an alcohol group, typically C₁-C₆, and neutralising with acetic acid.

The perfluoropolyether dialcohol, for example obtained by photo-oxidation (reaction with oxygen activated by UV radiation) of tetrafluoroethylene, contains a perfluoropolyethereal (Rf) chain with the following
structure:
-(CF₂-CF₂O)ₚ-(CF₂O)_{q} - where p/q = 0.5 - 3.0
with a molecular weight preferably comprised between 500 and 4000, more preferably between 1000 and 2000 and even more preferably comprised between 1400 and 1600.

For example, the perfluoropolyether (PFPE) of the PFPE-PU copolymer marketed by Solvay Solexis is suitable for technical applications (Fluorolink) and for cosmetic applications (Fomblin HC/PU-CAT5) under the INCI name "Polyurethane 26"), as an aqueous dispersion of 25% solids.

In certain embodiments, the cationic polyurethane used is perfluoropolyether-cationic polyurethane (Polyurethane-26).

According to certain embodiments, the system of the invention comprises the cationic polyurethane derivative component in quantities of 0.004 to 4% by weight, with respect to the total weight of the system.

In certain embodiments, the system of the invention may be incorporated in the formulation of non-rinse (leave-on) compositions or products, for example as preparations to be applied to the hair and scalp in the form of:
- water-based lotions such as solutions or aqueous colloidal dispersions
- hydroalcoholic lotions or solutions or hydroalcoholic colloidal dispersions
- fluid emulsions, oil in water emulsions
- hydrophilic gels, gelified aqueous or hydroalcoholic solutions formed by the addition of polymers (e.g. acrylic polymers such as Carbopol or high molecular weight Polyethylene glycols etc.) to the solvent.
- sera.

In other embodiments, the system of the invention may be incorporated in the formulation of rinse-off compositions or products, such as preparations to be applied to the hair and scalp in the form of:
- Shampoos or detergent systems containing surfactants;
- Balsams typically containing cationic substances (Polyquaternium, Cetyltrimethylammonium chloride, Docosyl trimethylammonium methyl sulphate etc.) and high-melting point alcohols or fatty acids to be applied to wet (or dry) hair before or after shampoo and then to be rinsed;
- Masks, typically containing cationic substances, in the form of gels.

In accordance with certain embodiments, the phospholipid system comprises
- 0.002-0.50% w/w hydroxypropyltrimonium hyaluronate
- 0.004-4.0% w/w polyurethane-26
- 0.005-5.0% w/w lecithin

The application of the system to the skin or keratinic structures results in the formation of a complex film, with hydrophobic and filmogenic characteristics.

Use of a cosmetic composition or preparation containing the phospholipid system according to one of the embodiments of the invention is then particularly advantageous since, by acting on the release kinetics of the functional substances or cosmetic agents contained therein, it allows, for the same efficacy, a reduction in the frequency of applications, thus improving user compliance towards the preparation.

Also disclosed herein is the simultaneous or separate use of a system according to any of claims 1-2 with a composition or cosmetic agent for preventing, avoiding or substantially reducing the removal of said composition or cosmetic agent from the keratinic structures of the body, including hair, epidermis and scalp, by means of or following the rinsing of said keratinic structures of the body with water.

### BRIEF DESCRIPTION OF THE FIGURES

The present invention shall be described in detail below and making reference to the figures, wherein:
Figure 1 shows the percentage of vellus hair in the comparative clinical study of example 7;
Figure 2 shows the percentage variation over T0 based on the clinical study reported in example 7.
Figure 3 shows a comparison of the absorbance spectra of the compositions of the invention and the negative control
Figure 4 shows a comparison of the absorbance spectra of the compositions of the invention, the negative control and the corresponding compositions of the same formulation but without the system of the invention and the control.
Figure 5 schematically shows the test results for the release of calcium pantothenate from compositions (solutions 1 and 2) with the system of the invention in various quantities and from a solution without the system.
Figures 6 A and B show the total percentage release of calcium pantothenate and the residual quantities on hair from compositions (solutions 1 and 2) with the system of the invention in various quantities and from a solution without the system.

The present invention shall now be described making reference to the following examples, which are provided purely by way of non-limiting illustration of the present invention.

### EXAMPLES

### Example 1

### SHAMPOO FOR TREATMENT OF ANDROGENIC ALOPECIA WITH SOYA LECITHIN

| Component (INCI name) | Quantity w/w (%) |
|---|---|
| Disodium Laureth Sulphosuccinate | 1.00-5.00 |
| Hydroxypropyltrimonium Hyaluronate | 0.005-0.50 |
| Polyurethane-26 | 0.004-4.0 |
| Lecithin (Glycine max L.) | 0.005-5.0 |
| Magnesium Laureth Sulphate | 5.00-9.00 |
| PEG-7 Glyceryl Cocoate | 0.50-1.00 |
| Cocamide MIPA | 0.50-2.00 |
| PEG-200 Hydrogenated Glyceryl Palmate | 0.50-2.00 |
| Polyquaternium-10 | 0.10-0.50 |
| Sodium Lauroyl Sarcosinate | 1.00-4.00 |
| Tetrasodium EDTA | 0.05-0.20 |
| BHA | 0.005-0.015 |
| Spermidine HCl | 0.001-0.15 |
| Biotin | 0.01-0.10 |
| Calcium pantothenate | 0.01-3.0 |
| Potassium Undecylenoyl Wheat Protein | 0.50-1.00 |
| Laureth-4 | 0.01-0.80 |
| Parfum | 0.10-0.80 |
| Glycol Distearate | 0.50-1.00 |
| Laureth-7 | 0.50-0.80 |
| Sodium Cocoamphoacetate | 0.05-3.00 |
| Cocamidopropyl Betaine | 0.01-2.00 |
| Sodium Laureth Sulphate | 0.01-3.00 |
| Sodium Hydroxymethylglycinate | 0.20-0.45 |
| Benzoic acid | 0.005-0.10 |
| Sodium hydroxide | as required |
| Citric acid | as required |
| Water | as required to 100.00 |

The lecithin used was of plant origin, obtained from Glycine max. L., and contained a phospholipid component with the following content:
80% by weight 3-sn-phosphatidylcholine
10% by weight 3-sn-Lysophosphatidylcholine
5% by weight Phosphatidylethanolamine
5% by weight Phosphatidic acid.

The lecithin used was supplied dissolved in ethyl alcohol and was stored at approx. 4°C.

### Example 2

### LOTION FOR TREATMENT OF ANDROGENIC ALOPECIA WITH SOYA LECITHIN

| Component (INCI name) | Quantity w/v (%) |
|---|---|
| Water | as required to 100 ml |
| Hydroxypropyltrimonium Hyaluronate | 0.005-0.50 |
| Polyurethane-26 | 0.004-4.0 |
| Lecithin (Glycine max L.) | 0.005-5.0 |
| Denatured alcohol | 15.0-20.0 |
| Spermidine HCl | 0.005-0.15 |
| Biotin | 0.01-0.10 |
| Calcium pantothenate | 0.1-3.0 |
| Rutin | 0.001-0.05 |
| PEG-40 Hydrogenated Castor Oil | 0.5-2.0 |
| Octadecyl Di-t-butyl-4-hydroxyhydrocinnamate | 0.05 |
| Parfum | 0.20 |
| Zeaxanthin | 0.002-0.01 |
| Helianthus annuus seed oil | 0.001-0.01 |
| Lactic acid | as required to pH 5.0 |

### Example 3

### BODY WASH WITH DIPOTASSIUM AND AMMONIUM GLYCYRRHIZATE

| Component (INCI name) | Quantity w/w (%) |
|---|---|
| Water | as required to 100 g |
| Sodium Laureth Sulphate Aqua | 9-15% |
| Ammonium Laureth sulphate | 1-3% |
| Disodium Cocoamphodiacetate | 3-5% |
| Sodium Cocoyl Glutamate | 3-6 % |
| Monoammonium glycyrrhizate | 1.8% |
| Dipotassium glycyrrhizate | 0,2 % |
| Hydroxypropyltrimonium Hyaluronate | 0.005-0.50 |
| Polyurethane-26 | 0.004-4.0 |
| Lecithin (Glycine max L.) | 0.005-5.0 |
| PEG-150 Pentaerythrityl Tetrastearate | 1-3% |
| PEG-6 Caprylic/Capric Glycerides | 0.5-3% |
| Lactic acid | as required to pH 6.0 |
| Methylparaben | 0.05-0.2 |
| Propylparaben | 0.05-0.2 |
| Phenoxyethanol | 0.5-0.8% |
| Parfum (Allergen Free) | |

### Example 4

### LOTION FOR TREATMENT OF ANDROGENIC ALOPECIA WITH LECITHIN

| Component (INCI name) | Quantity w/v (%) |
|---|---|
| Water | as required to 100 ml |
| Hydroxypropyltrimonium Hyaluronate | 0.005-0.50 |
| Polyurethane-26 | 0.004-4.0 |
| Lecithin (LECITHIN 2) | 0.005-5.0 |
| Denatured alcohol | 15.0-20.0 |
| Spermidine HCl | 0.005-0.15 |
| Biotin | 0.001-0.10 |
| PEG-40 Hydrogenated Castor Oil | 0.5-2.0 |
| Octadecyl Di-t-butyl-4-hydroxyhydrocinnamate | 0.05 |
| Parfum | 0.20 |
| Helianthus annuus seed oil | 0.001-0.01 |
| Lactic acid | as required to pH 5.0 |

The soya lecithin used had a content of 94% by weight phosphatidylcholine and 6% by weight lyso-phosphatidylcholine.

### Example 5

### HAIR-CARE SERUM

| Component (INCI name) | Quantity w/v (%) |
|---|---|
| Water | as required to 100 ml |
| Hydroxypropyltrimonium Hyaluronate | 0.005-0.50 |
| Polyurethane-26 | 0.004-4.0 |
| Lecithin (Glycine max L.) | 0.005-5.0 |
| Denatured alcohol | 15.0-20.0 |
| Spermidine HCl | 0.005-0.15 |
| Potassium Octatrienoate | 0.001-0.20 |
| Biotin | 0.01-0.10 |
| Calcium pantothenate | 0.1-3.0 |
| Ajuga reptans leaf extract | 0.001-0.1 |
| Fermented soya (Glycine max L.) | 0.001-0.5 |
| Hydrogenated Castor oil | 0.50-0.9 |
| Octadecyl Di-t-butyl-4-hydroxyhydrocinnamate | 0.05 |
| Parfum | |
| Ethoxydiglycol | 0.05-1.0 |
| Hydroxypropyl guar | 0.1-0.8 |
| Lactic acid | as required to pH 5.0 |

### Example 6

### CONDITIONING BALSAM WITH LECITHIN

| Component (INCI name) | Quantity w/w (%) |
|---|---|
| Water | as required to 100 g |
| Cetearyl Alcohol | 0.5-4.0 |
| Hydroxypropyltrimonium Hyaluronate | 0.005-0.50 |
| Polyurethane-26 | 0.004-4.0 |
| Lecithin (Glycine max L.) | 0.005-5.0 |
| Palmitic acid | 0.5-4.0 |
| Myristic acid | 0.5-4.0 |
| Hydrolysed Wheat Protein | 0.05-1.00 |
| Cetrimonium Chloride | 1.0-3.0 |
| Behentrimonium Methosulphate | 0.5-3.0 |
| Panthenol | 0.5-3.0 |
| Pentylene glycol | 5.0 |
| Spermidine HCl | 0.005-0.15 |
| Phenoxyethanol | 1.0 |
| Perfume | as required |

The lecithin used had a phosphatidylcholine content of 96% by weight and a lyso-phosphatidylcholine content of 4% by weight.

### Example 7

A randomised, double-blind clinical study has been conducted to evaluate the efficacy of a hair-care lotion with the modified release system of Example 2. The lotion is applied to the scalp of subjects twice per week, compared to daily application of a lotion with the same composition but without the phospholipid system of the invention.

120 subjects of both sexes affected by telogen defluvium have participated in the study, divided into 3 groups and subjected to treatment with:
1. lotion with novel release twice per week (group 1)
2. lotion without the release technology applied daily (group 2)
3. placebo lotion (without the functional active substances and without the release technology) (group 3).

Objective instrumental and dermatological evaluations have been conducted on inclusion of the subjects in the study (T0), after 1 month of treatment (T30), after 2 months of treatment (T60) and after 3 months of treatment (T90).

The subjects recruited should not have had systemic or topical therapy for hair loss in the three months prior to the study. Subjects affected by dermatological or systemic pathologies or undergoing therapy with specific medicinal products have also been excluded.

Efficacy data for the lotion is summarised below.

In particular, the % of vellus hair has been evaluated

| | T0 | T30 | T60 | T90 |
|---|---|---|---|---|
| | (mean %) | (mean %) | (mean %) | (mean %) |
| Group 1 | 6.08 | 5.13 | 4.98 | 4.67 |
| Group 2 | 5.26 | 4.50 | 4.38 | 4.21 |
| Group 3 | 4.51 | 4.45 | 4.48 | 4.59 |

While the % variation over T0 is summarised in the Table below

| | T30 (mean %) | T60 (mean %) | T90 (mean %) |
|---|---|---|---|
| Group 1 | - 15.62 | - 18.09 | - 23.19 |
| Group 2 | - 14.45 | - 16.73 | - 19.96 |
| Group 3 | - 1.3 | - 0.66 | + 1.77 |

The preliminary data obtained has made it possible to highlight the efficacy of the novel technology applied to an anti-hair loss lotion. The observed effect was greater than that obtained for the lotion without the novel technology.

The contents of Italian patent application MI2011A000644 of 14/04/2012 is claimed herein and integrated in the present description in its entirety.

### Example 8

A study procedure has been created to verify the efficacy resulting from use of a system for the release of cosmetic agents of the invention, simulating treatment with a cosmetic composition containing the system in the laboratory and verifying resistance to a repeated wash cycle in comparison to a cosmetic composition (comparator) having the same formulation but without said system.

### 1. MATERIALS

### 1.1. Formulations tested

The formulations reported below have been prepared:
System 1

| Component (INCI name) | % w/v |
|---|---|
| Hyd roxypropyltrimonium Hyaluronate | 0.071 |
| Denatured alcohol | 10.750 |
| Calcium pantothenate | 1.671 |
| Lecithin (Glycine max L.), Alcohol | 0.050 |
| Polyurethane-26 | 0.020 |
| Water | as required to 100 ml |

System 2

| Component (INCI name) | % w/v |
|---|---|
| Hyd roxypropyltrimonium Hyaluronate | 0.500 |
| Denatured alcohol | 10.750 |
| Calcium pantothenate | 1.671 |
| Lecithin (Glycine max L.), Alcohol | 0.050 |
| Polyurethane-26 | 4.000 |
| Water | as required to 100 ml |

In order to detect release of the aforementioned formulations spectrophotometrically, all formulations tested have been supplemented with the same percentage (1.67%) of calcium pantothenate (absorbance peak between 190 and 200 nm).

This way it has been possible to obtain relative quantification of release of the formulation following washes.

### 1.2. Positive controls (system 1 and 2)

In order to exclude any absorbance from other constituents of the formulation containing the system (1 and 2) of the invention, the same formulations without calcium pantothenate have been prepared in parallel according to the following formulations:

| Component (INCI name) | % w/v |
|---|---|
| Hyd roxypropyltrimonium Hyaluronate | 0.071 |
| Denatured alcohol | 10.750 |
| Lecithin (Glycine max L.), Alcohol | 0.050 |
| Polyurethane-26 | 0.020 |
| Water | as required to 100 ml |

| Component (INCI name) | % w/v |
|---|---|
| Hyd roxypropyltrimonium Hyaluronate | 0.500 |
| Denatured alcohol | 10.750 |
| Lecithin (Glycine max L.), Alcohol | 0.050 |
| Polyurethane-26 | 4.000 |
| Water | as required to 100 ml |

### 1.3. Negative controls (solutions without the technology)

Two formulations containing water and ethanol or water, ethanol and calcium pantothenate respectively have been prepared as negative controls for the experiment (no system of the invention).

| Component (INCI name) | % w/v |
|---|---|
| Denatured alcohol | 10.750 |
| Water | as required to 100 ml |

| Component (INCI name) | % w/v |
|---|---|
| Denatured alcohol | 10.750 |
| Calcium pantothenate | 1.671 |
| Water | as required to 100 ml |

### 1.4. Study model

6 locks of untreated donor hair, arranged into locks having the same weight (approx. 2.9 g).

### 2. EXPERIMENTAL PROCEDURE

2.1. Day 1: treatment of locks with the different formulations and the positive and negative controls
   1. Preparation, in locks of approx. 2.9 g each, of 6 untreated donor lock samples;
   2. Dispensing of 30 mL of each formulation and the corresponding positive and negative controls, into suitably labelled sterile Falcon tubes;
   3. Immersion of the locks into the corresponding formulations and the controls with the aid of forceps. The locks have been completely immersed in the formulations for a time of 3 minutes;
   4. Incubation, on expiry of the 3 minutes. The locks have been removed from the Falcon tubes (with the aid of forceps) allowed to drip roughly into the Falcon tubes and hung to dry for 2 minutes;
   5. Treatment of the dried locks using a warm air hair-drier for 15 minutes and subsequent resting overnight at room temperature to complete drying;
2.2. DAY 2-3: WASHING OF THE LOCKS AND SPECTROPHOTOMETRIC READING
   The locks, now dry, have been subjected to 4 sequential washes in distilled water, according to the procedure described below:
   1. For each formulation and for the corresponding positive and negative controls, 4 Falcon tubes, containing 30 mL of distilled water, have been prepared and suitably labelled for washing;
   2. Each lock has been fully immersed for 1 minute in the first wash Falcon tube (with the aid of forceps);
   3. On completion of the incubation in distilled water, the locks have been held vertically and allowed to drip into the same wash Falcon tube until dripping has finished;
   4. The locks have then been immersed in the subsequent wash, according to the same previously described method;
   5. The procedure has been repeated for washes 3 and 4;
   6. The locks have been once more positioned to drip dry;
   7. The wash fluids have been subjected to spectrophotometric analysis.
      - Spectrophotometric readings have been conducted using a Jasco model V-530 spectrophotometer;
      - Readings have been performed using a quartz cuvette according to the following conditions: wavelength range equal to 190-280 nm, 200 nm/min, reading interval 0.1 nm;
      - Each solution read has been diluted 50 fold in distilled water in a final cuvette volume equal to 2 mL;
      - Each reading has been performed in duplicate;
      - Preliminary readings of the formulations (at the same dilution) and the negative and positive controls have been performed prior to immersion of the locks in order to obtain the maximum absorbance peak of the aforementioned formulation and subsequently determine the % of calcium pantothenate released following each wash.
2.3. DATA PROCESSING
   On completion of the spectrophotometric readings, the absorbance spectra obtained have been processed using the software package "Essential eFTIR".
   2.3.1. Validation of the experimental method used:
      1. The absorbance spectra of the formulations as they are and the negative control containing calcium pantothenate have been compared with the corresponding formulations and controls without said substance;
      2. For each comparison, the maximum peak corresponding to the absorbance of calcium pantothenate has been selected;
   2.3.2. Evaluation of calcium pantothenate release
      3. The absorbance spectra of the formulations as they are and the positive and negative controls containing calcium pantothenate have been compared with the absorbance spectra of the corresponding washes (4 washes);
      4. The maximum peak corresponding to the absorbance of calcium pantothenate for the formulations as they are and for the corresponding washes has been selected for each comparison;

### 3. RESULTS

### 3.1.1. Validation of the experimental method used:

Comparison of the negative absorbance spectra containing calcium pantothenate with the corresponding control without said substance has made it possible to determine the validity of the experimental method used.

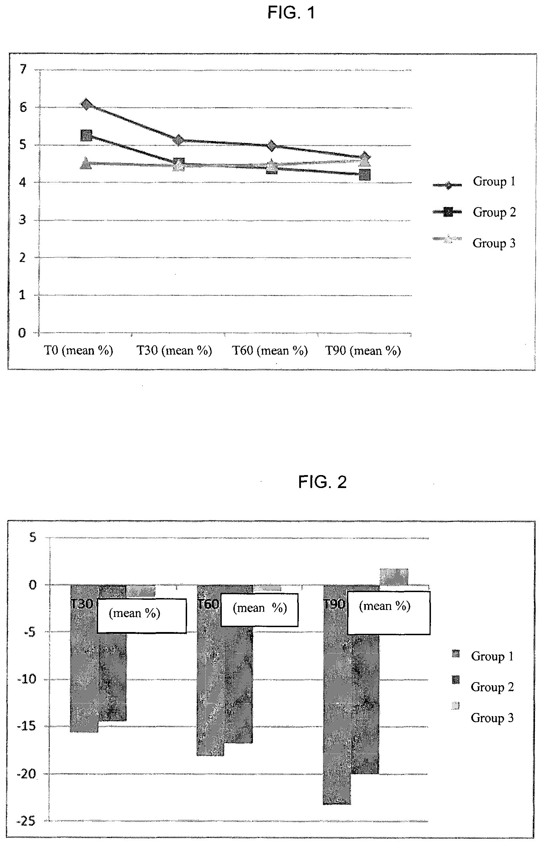

As is clearly visible in figure 1, the absorbance peak is only recorded around 200 nm, and only in the control containing calcium pantothenate.

Based on the present data, the absorbance spectra of the formulations as they are containing calcium pantothenate have been compared with the corresponding formulations and controls without said substance.

Again in this case, an absorbance peak has only been recorded around 200 nm and only in the formulations containing calcium pantothenate.

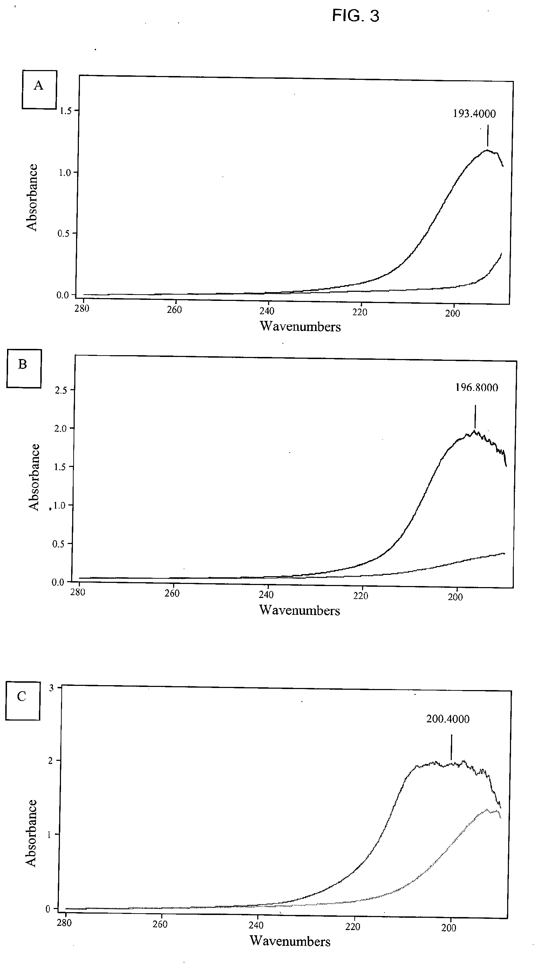

Hence, using the experimental procedure described, it is possible to obtain a clear determination of the quantity of formulation released from locks treated with the various formulations following sequential washing in distilled water. Said quantity shall be expressible in terms of the % of calcium pantothenate released. Figure 3 highlights the results of the comparison of the absorbance spectra of the formulations as they are and of the negative control containing calcium pantothenate and the corresponding formulations and controls without said substance. (A) Negative control; (B) Formulation System 1; (C) Formulation System 2.

### 3.1.2. Evaluation of calcium pantothenate release:

The absorbance spectra of the formulations as they are and the negative control containing calcium pantothenate have been compared with the absorbance spectra of the corresponding washes (4 washes).

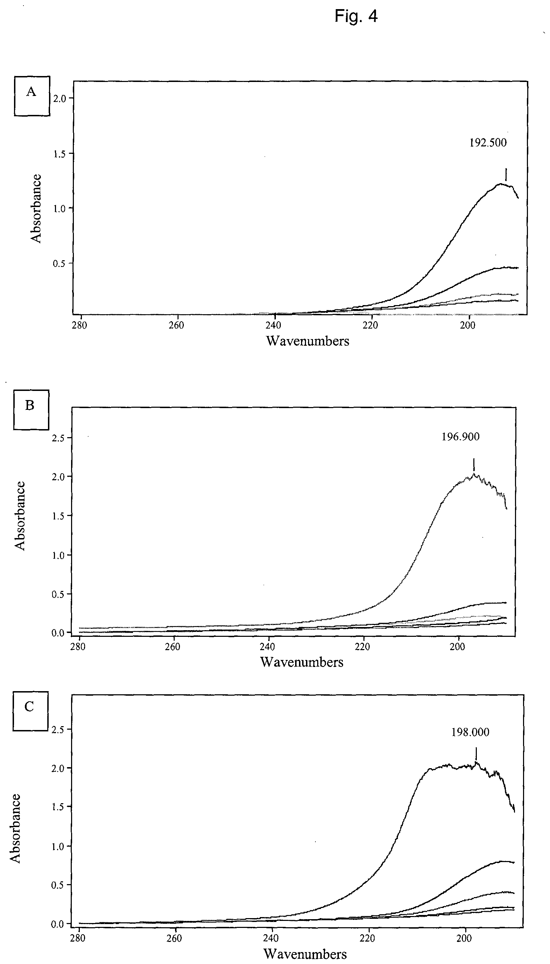

The maximum peak corresponding to the absorbance of calcium pantothenate for the formulations as they are and for the corresponding washes has been selected for each comparison (Fig. 4).

In particular, Figure 4 shows the comparison of the absorbance spectra of the formulations as they are, the negative control containing calcium pantothenate and the corresponding washes. (A) Negative control; (B) Formulation System 1; (C) Formulation System 2.

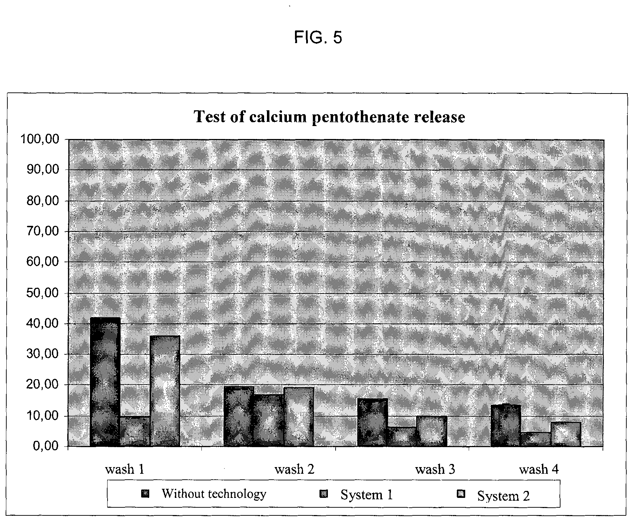

From analysis of the spectrophotometric peaks of the corresponding washes compared with the peak of the formulation as it is (prior to immersion of the lock) it has been possible to quantify resistance to washing by means of indirect analysis of the % release of calcium pantothenate (Fig. 5/Test of release of calcium pantothenate).

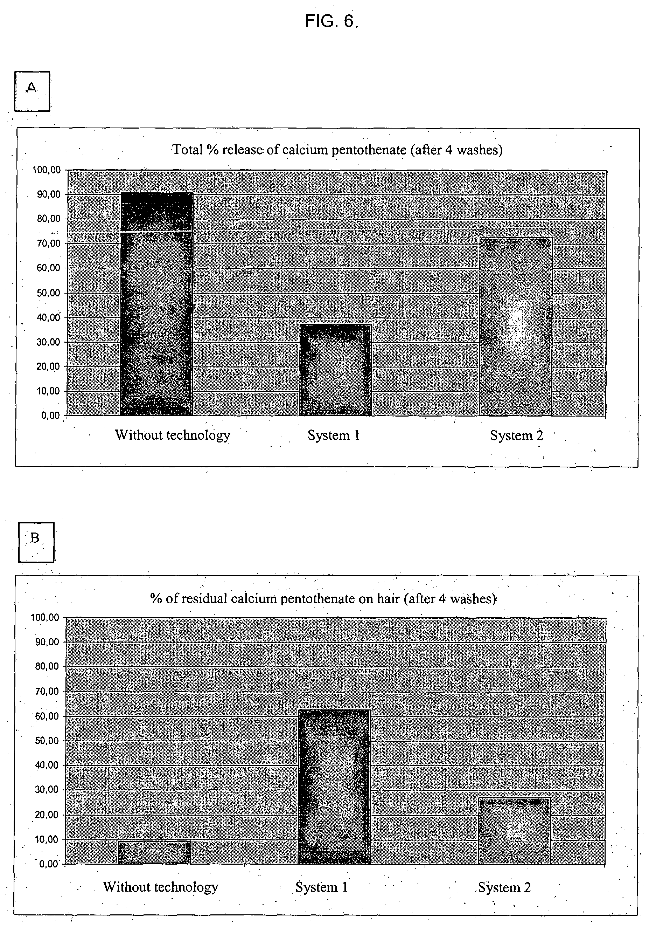

Specifically, Figure 5 shows the percentage of calcium pantothenate released by the solution without the system, by the solution with system 1 and by the solution with system 2, with respect to the 4 sequential washes. The data is expressed as the mean % ± SD with respect to the calcium pantothenate in the formulations prior to immersion of the lock. Each experiment has been conducted in duplicate. The results of total % release after the 4 washes for the formulations tested are reported in Figures 6 A and B.

Specifically, Figure 6 A shows the total percentage of calcium pantothenate released by the solution without the system, by the solution with system 1 and by the solution with system 2 respectively, after the 4 sequential washes. Figure 6 B shows the total percentage of residual calcium pantothenate in the lock treated with the solution without the system, with the solution with system 1 and with the solution with system 2 respectively, after the 4 sequential washes.

The data is expressed as the mean % ± SD with respect to the calcium pantothenate in the formulations prior to immersion of the lock. Each experiment has been conducted in duplicate.

The data shows a marked difference in washing away with the formulation with system 1 and with the formulation with system 2.

In conclusion, it has been observed that:
- following rinsing, the cosmetic composition without the system loses over 90% of the activity (calcium pantothenate) overall;
- the cosmetic composition containing system 1 loses only 37% of activity overall;
- the cosmetic composition containing system 2 loses only 72% of activity overall.

## Claims

1. System for the release of cosmetic agents **characterised in that** it comprises
i) at least one phospholipid,
ii) hyaluronic acid 2-hydroxy-3-(N,N,N-trimethylammonium) propyl chloride,
iii) a cationic polyurethane derivative being a perfluoropolyether-polyurethane (PFPE-PU) copolymer comprising a perfluoropolyethereal (Rf) chain having the structure:
-(CF2-CF2O)p-(CF2O)q-
wherein p/q = 0.5 - 3.0,
wherein said perfluoropolyethereal (Rf) chain has a molecular weight of 500 to 4000, preferably of 1000 to 2000 and more preferably of 1400 to 1600,
in cosmetically acceptable amounts.

2. System according to claim 1 **characterised in that** said phospholipid comprises a phospholipid fraction selected from phosphatidylcholine, phosphatidylserine, phosphatidylethanolamine, phosphatidylinositol, dipalmitoylphosphatidylcholine, lysophospholipids, zwitterionic phospholipids, sphingomyelin, sphingolipids, glycerophospholipids, sphingoglycolipids or mixtures thereof.

3. Cosmetic composition comprising at least one cosmetically active ingredient, one or more excipients or carriers and a system according to any one of claims 1-2.

4. Use of a cosmetic composition according to claim 3, as a non-rinse (leave-on) composition for hair and scalp selected from
- water-based lotions,
- hydroalcoholic lotions,
- fluid emulsions,
- hydrophilic gels,
- sera.

5. Use of a cosmetic composition according to claim 4, **characterised in that** said
- water-based lotions are selected from solutions or aqueous colloidal dispersions,
- hydroalcoholic lotions are solutions or hydroalcoholic colloidal dispersions,
- fluid emulsions are emulsions of oil in water or water in oil,
- hydrophilic gels are gelified aqueous or hydroalcoholic solutions formed by the addition of polymers to a solvent,
- said sera are alcoholic or hydroalcoholic serous solutions.

6. Use of a system according to claim 1 to make an agent or a cosmetic composition adhesive to the epidermis or a keratinic structure of the human body.

## Patentansprüche

1. System zur Abgabe kosmetischer Agentien, **dadurch gekennzeichnet, dass** es aufweist:
i) zumindest ein Phospholipid,
ii) Hyaluronsäure 2-hydroxy-3-(N,N,N-trimethylammonium) Propylchlorid,
iii) ein kationisches Polyurethan-Derivat, das ein Perfluorpolyetheröl-Polyurethan (PFPE-PU) - Copolymer ist, das eine Perfluorpolyetheröl-Kette (Rf) mit der Struktur
-(CF2-CF2O)p-(CF2O)q-
aufweist,
wobei p/q = 0,5 - 3,0 ist,
wobei die Perfluorpolyetheröl-Kette (Rf) ein Molekulargewicht von 500 bis 4.000 aufweist, bevorzugt 1.000 bis 2.000 und weiter bevorzugt 1.400 bis 1.600,
in kosmetisch akzeptablen Mengen.

2. System gemäß Anspruch 1, **dadurch gekennzeichnet, dass** das Phospholipid eine Phospholipidfraktion aufweist, ausgewählt aus Phosphatidylcholin, Phosphatidylserin, Phosphatidylethanolamin, Phosphatidylinositol, Dipalmitoylphosphatidylcholin, Lysophospholipide, zwitterionische Phospholipide, Sphingomyelin, Sphingolipide, Glycerophospholipide, Sphingoglycolipide oder Mischungen davon.

3. Kosmetische Zusammensetzung, aufweisend zumindest einen kosmetischen Wirkstoff, einen oder mehrere kosmetische Hilfsstoffe oder Trägerstoffe und ein System gemäß einem der Ansprüche 1 oder 2.

4. Verwendung einer kosmetischen Zusammensetzung gemäß Anspruch 3 als nichtabzuspülende (verbleibende) Zusammensetzung für Haare und Kopfhaut, ausgewählt aus
- wasserbasierten Lotionen,
- wässrig-alkoholischen Lotionen,
- flüssigen Emulsionen,
- hydrophilen Gels,
- Seren.

5. Verwendung einer kosmetischen Zusammensetzung gemäß Anspruch 4, **dadurch gekennzeichnet, dass**
- die wasserbasierten Lotionen ausgewählt sind aus Lösungen oder wässrigen kolloidalen Dispersionen,
- die wässrig-alkoholischen Lotionen Lösungen oder wässrige kolloidale Dispersionen sind,
- die flüssigen Emulsionen Emulsionen von Öl in Wasser oder Wasser in Öl sind,
- die hydrophilen Gels gelierte wässrige oder wässrig-alkoholische Lösungen sind, die durch Zugabe von Polymeren zu einem Lösungsmittel gebildet wurden,
- die Seren alkoholische oder wässrig-alkoholische seröse Lösungen sind.

6. Verwendung eines Systems gemäß Anspruch 1 zur Herstellung eines Agens oder einer kosmetischen Zusammensetzung, die auf der Haut oder einer Keratinstruktur des menschlichen Körpers haftet.

## Revendications

1. Système de libération d'agents cosmétiques **caractérisé en ce qu'**il comprend
i) au moins un phospholipide,
ii) du 2-hydroxy-3-(N,N,N-triméthylammonium)propyl chlorure d'acide hyaluronique,
iii) un dérivé de polyuréthane cationique étant un copolymère de perfluoropolyéther-polyuréthane (PFPE-PU) comprenant une chaîne perfluoropolyéthérée (Rf) ayant la structure :
-(CF2-CF2O)p-(CF2O)q-
dans lequel p/q = 0,5 à 3,0,
dans lequel ladite chaîne perfluoropolyéthérée (Rf) a une masse moléculaire de 500 à 4 000, de préférence de 1 000 à 2 000 et de manière davantage préférée de 1 400 à 1 600,
en des quantités cosmétiquement acceptables.

2. Système selon la revendication 1 **caractérisé en ce que** ledit phospholipide comprend une fraction de phospholipide sélectionnée parmi la phosphatidylcholine, la phosphatidylsérine, la phosphatidyléthanolamine, le phosphatidylinositol, la dipalmitoylphosphatidylcholine, les lysophospholipides, les phospholipides zwittérioniques, la sphingomyéline, les sphingolipides, les glycérophospholipides, les sphingoglycolipides ou les mélanges de ceux-ci.

3. Composition cosmétique comprenant au moins un principe cosmétiquement actif, un ou plusieurs excipients ou véhicules et un système selon l'une quelconque des revendications 1 et 2.

4. Utilisation d'une composition cosmétique selon la revendication 3, en tant que composition sans rinçage (qui reste) pour les cheveux et le cuir chevelu sélectionnée parmi
- des lotions à base d'eau,
- des lotions hydroalcooliques,
- des émulsions fluides,
- des gels hydrophiles,
- des sérums.

5. Utilisation d'une composition cosmétique selon la revendication 4, **caractérisée en ce que** lesdits
- lotions à base d'eau sont sélectionnées parmi des solutions ou des dispersions colloïdales aqueuses,
- lotions hydroalcooliques sont des solutions ou des dispersions colloïdales hydrocalcooliques,
- émulsions fluides sont des émulsions huile-dans-l'eau ou eau-dans-l'huile,
- gels hydrophiles sont des solutions aqueuses ou hydroalcooliques gélifiées formées par l'ajout de polymères à un solvant,
- sérums sont des solutions alcooliques ou hydroalcooliques séreuses.

6. Utilisation d'un système selon la revendication 1 pour rendre un agent ou une composition cosmétique adhésif à l'épiderme ou à une structure kératinique du corps humain.
